# EUROPEAN PATENT SPECIFICATION

(11) **EP 4 452 195 B1**
(45) Date of publication and mention of the grant of the patent: **12.02.2025**
(21) Application number: 22834970.0
(22) Date of filing: 09.12.2022
(51) Int. Cl.: A61K 8/31, A61K 8/34, A61K 8/37, A61K 8/04, A61Q 15/00

(54) **COSMETIC SWEAT MANAGEMENT COMPOSITIONS**
KOSMETISCHE SCHWEISSREGULIERENDE ZUSAMMENSETZUNGEN
COMPOSITIONS COSMÉTIQUES POUR LA REGULATION DE LA SUEUR

(30) Priority: 22.12.2021 EP 21217099
(43) Date of publication of application: 30.10.2024
(73) Proprietor: Unilever IP Holdings B.V., 3013 AL Rotterdam (NL); UNILEVER GLOBAL IP LIMITED, Wirral Merseyside CH62 4ZD (GB)
(72) Inventor: CROPPER, Martin Peter, 6708 WH Wageningen (NL); FLETCHER, Neil Robert, 6708 WH Wageningen (NL); JONES, Kimberley Ann, 6708 WH Wageningen (NL); MUNIER, Veronique Sandrine, 6708 WH Wageningen (NL); STOCKTON, Joanne Elizabeth, 6708 WH Wageningen (NL)
(74) Representative: Whaley, Christopher
(86) International application number: PCT/EP2022/085123
(87) International publication number: WO 2023/117480

(56) References cited:
- WO-A1-2020/108878
- WO-A1-2020/108886
- US-A1- 2009 130 042

## Description

### Field of Invention

The present invention is in the field of cosmetic compositions and their use as sweat management products, in particular, non-aluminium sweat management products.

### Background

EP 550,960 A1 (Unilever, 1992) discloses the use as an antiperspirant active of a lipid mixture which forms, upon contact with perspiration, a water-insoluble liquid crystal phase of greater than one dimensional periodicity. This publication does not disclose ethanolic compositions, nor their stability issues, nor their use in aerosol compositions.

WO 94/024993 (Unilever, 1994) discloses an antiperspirant composition comprising a lipid mixture which forms, upon contact with perspiration, a water-insoluble liquid crystal phase of greater than one dimensional periodicity, in a cosmetic vehicle comprising a volatile silicone and containing less than 10% by weight of the total composition of a short chain monohydric alcohol. This publication does not disclose compositions with the specific ratios of components as defined herein and does not disclose their importance in the low temperature (0°C) stability thereof.

U2009/130042 A1 (Unilever, 2009) discloses cosmetic compositions comprising isostearyl alcohol, glycerol monolaurate and ethanol, but does not disclose such compositions additionally comprising a C8-C16 alkane.

WO2020/108878 A1 (Unilever, 2020) discloses cosmetic compositions comprising isostearyl alcohol, glycerol monolaurate and ethanol, but does not disclose such compositions additionally comprising a C8-C16 alkane.

WO2020/108882 A1 (Unilever, 2020) discloses antiperspirant aerosol compositions comprising ethanol, amphiphilic material similar to that disclosed in the above publications and volatile silicone, the ratios and amounts of the components being selected to provide good storage stability for the aerosols, particularly at low temperatures.

WO2020/108885 A1 (Unilever, 2020) discloses antiperspirant compositions comprising ethanol, isostearyl alcohol, glycerol monolaurate, volatile silicone and humectant, the ratios and amounts of the components being selected to provide good storage stability.

WO2020/108886 A1 (Unilever, 2020) discloses cosmetic compositions comprising isostearyl alcohol, glycerol monolaurate and ethanol, but does not disclose such compositions additionally comprising a C8-C16 alkane

EP 3,773,433 B1 (Unilever, 2021) discloses antiperspirant compositions comprising ethanol, isostearyl alcohol, glycerol monolaurate and volatile silicone, the ratios of the components being selected to provide good storage stability, particularly at low temperatures.

### Summary of Invention

It is an object of the present invention to provide a cosmetic composition that does not require the presence of an aluminium salt to deliver a sweat management benefit. It is a further object of the present invention to do this from a composition that has a high degree of storage stability, particularly at low temperatures.

It is an object of the present invention to provide a cosmetic sweat management composition that is free from silicone oil and which has good stability, particularly at low temperature. It is a further object of the present invention to provide such a cosmetic aerosol composition that does not require the presence of an aluminium salt to deliver an antiperspirancy benefit.

In a first aspect of the invention, there is provided a cosmetic composition comprising:
(i) 24.6 to 95% ethanol;
(ii) 2 to 18% of a lipid mixture consisting of ISA and GML at a weight ratio of from 25: 75 to 40: 60;
(iii) 3 to 70% of an oil selected from C8-C16 alkanes,
wherein (a) the amount indicated for component (i) includes any fragrance present in the composition; (b) the amounts indicated for components (i) to (iii) exclude any volatile propellant present in the composition; and (c) wherein the content of component (ii) is less than or equal to the content of component (i) multiplied by 0.22.

In a further aspect of the invention, there is provided a non-therapeutic cosmetic method of attaining a sweat management benefit comprising the topical application of a composition according to the first aspect of the invention.

### Detailed Description

Herein, features expressed as "preferred" with regard to a particular aspect of the invention should be understood to be preferred with regard to each aspect of the invention (likewise, features expressed as "more preferred" or "most preferred").

Herein, preferred features of the invention are particularly preferred when used in combination with other preferred features.

Herein, "ambient conditions" refers to 25°C and 1 atmosphere pressure, unless otherwise indicated.

Herein, the term "volatile" refers to a material having a boiling point of less 10°C.

Herein, all percentages, ratios and amounts are by weight, unless otherwise indicated. Herein, the word "comprising" is intended to mean "including" but not necessarily "consisting of", i.e., it is non-exhaustive.

Herein, "cosmetic" methods and compositions should be understood to mean non-therapeutic methods and compositions, respectively.

Herein, "water-insoluble" means having a solubility in water of less than 0.1% by weight (at 37°C).

The compositions of the invention are particularly effectively when applied to the underarm regions of the human body and/or the feet. The compositions are especially effectively when applied to the underarm regions of the human body.

Cosmetic aerosol compositions consist of a propellant and a base. The components of the base are typically mixed together first and the propellant is added last in a process sometimes called "gassing".

Herein, the "base" of a cosmetic aerosol composition is all the components of the total composition other than the propellant.

It is important that the fully formulated cosmetic aerosol composition has good storage stability, so that it can survive prolonged transit to stores and extended periods on shelf prior to purchase and use.

The present invention involves compositions having superior storage stability, particularly at low temperatures, such as 0°C. By achieving stability at low temperatures, the present invention enables effective transit and storage of the claimed compositions in cold regions of the world. In addition, it enables transit and storage at refrigerated temperatures as may be required for the preservation of heat sensitive ingredients of the composition, such as certain fragrance components.

The lipid mixture is a mixture of isostearyl alcohol and glycerol monolaurate at a weight ratio of from 25: 75 to 45: 55. This lipid mixture is able to form an inverse hexagonal phase following contact with sweat on the surface of the human body and this leads to the antiperspirancy benefit of the compositions of the invention (as disclosed in EP 550,960 A1 [Unilever, 1992]).

The lipid content of the composition is from 2% up to a theoretical maximum of 18%, ignoring any propellant present in the composition; however, the maximum lipid content is also limited to the weight percentage of ethanol in the composition multiplied by 0.22. Hence, a lipid content of 18% can only be achieved when the ethanol content is relatively high. The ethanol serves as a solvent for the lipid; hence the interdependency of their levels.

The content of lipid mixture in the composition, excluding any propellant therein, is preferably at least 4%, more preferably at least 5% and most preferably at least 10%. The content of lipid mixture, again ignoring and propellant in the composition, is preferably 30% or less and more preferably 18% or less and most preferably 15% or less, these upper amounts being combinable with each of the lower ranges indicated in this paragraph to give preferred ranges of incorporation.

The purpose of the ethanol is principally to solubilise the lipid mixture. The ethanol is present at from 24.6 to 95% of the composition, excluding any propellant therein.

The content of ethanol in the composition, ignoring the propellant therein, is preferably at least 25%, more preferably at least 15% and most preferably at least 30%. The content of ethanol, again ignoring and propellant in the composition, is preferably 90% or and more preferably 75% or less, these upper amounts being combinable with each of the lower ranges indicated in this paragraph to give preferred ranges of incorporation.

Preferred composition according to the invention are aerosol compositions. Such compositions typically employ a volatile propellant. The propellant is commonly either a compressed gas or a material that boils at below ambient temperature, preferably at below 0°C, and especially at below -10°C. Examples of compressed gasses include compressed air, nitrogen and carbon dioxide. Examples of suitable propellants include volatile hydrocarbons, dimethyl ether and hydrofluorocarbons containing from 2 to 4 carbons, at least one hydrogen and 3 to 7 fluorine atoms. In particularly preferred embodiments, the propellant used comprises or is solely a hydrocarbon propellant.

Preferred hydrocarbons for use as propellant include propane, butane, isobutane and mixtures thereof.

When compositions according to the invention are aerosol compositions, they can be made in a conventional manner by first preparing a base composition, charging the base composition into the aerosol can, fitting a valve assembly into the mouth of the can, thereby sealing the can, and thereafter charging propellant into the can to a desired pressure, and finally fitting an actuator on or over the valve assembly.

When employed, propellant is included in the total composition at a level of from 20 to 95% and preferably at a level of from 50 to 90%.

Cosmetic compositions according to the present invention are preferably free from aluminium or zirconium salts.

A preferred additional component for use in compositions of the present invention is a fragrance or fragrance oil, sometimes alternatively called a perfume (oil). The fragrance oil may comprise a single fragrance or component more commonly a plurality of fragrance components. Herein, fragrance oils impart an odour, preferably a pleasant odour, to the composition. Preferably, the fragrance oil imparts a pleasant odour to the surface of the human body the composition is applied to the same.

The amount of fragrance oil in the composition is commonly up to 3% advantageously is at least 0.5% and particularly from 0.8% to 2%.

A preferred additional component of compositions of the invention is a deodorant active. These are typically antimicrobial agents active against bacterial on the skin of the human body. These serve to reduce malodour and especially useful in compositions in which the lipid mixture is not itself an antimicrobial agent. When employed, the level of incorporation is preferably 0.01%-5%, more preferably from 0.01-2% and most preferably from 0.03%-0.5% by weight of the total composition.

Preferred anti-microbial deodorant agents are those that are more efficacious than simple alcohols such as ethanol. Particularly preferred anti-microbial deodorant agents are soluble in ethanol, meaning that they a solubility in ethanol of at least 10g/L at 20°C. Examples of suitable anti-microbial deodorant agents include niacinamide; quaternary ammonium compounds, like cetyltrimethylammonium salts; chlorhexidine and salts thereof; and diglycerol monocaprate, diglycerol monolaurate, glycerol monolaurate, and similar materials, as described in "Deodorant Ingredients", S.A.Makin and M.R.Lowry, in "Antiperspirants and Deodorants", Ed. K. Laden (1999, Marcel Dekker, New York). More preferred are polyhexamethylene biguanide salts (also known as polyaminopropyl biguanide salts), an example being Cosmocil CQ available from Arch Chemicals, 2',4,4'-trichloro,2-hydroxy-diphenyl ether (triclosan), 3,7,11-trimethyldodeca-2,6,10-trienol (farnesol), essential oils such as Tea Tree Oil and Thyme Oil, climbazole, octapyrox, ketoconazole, zinc pyrithione and mixtures thereof.

A preferred optional component is a preservative, such as ethyl or methyl parabens or BHT (butyl hydroxy toluene), typically in an amount of from 0.01 to 0.1% by weight of the total composition.

The invention will now be described by way of some examples.

### Examples

Numerous examples and comparative examples have been prepared and tested. Without exception, the Examples according to the invention had superior stability to the Comparative Examples.

The compositions detailed in Table 1 were prepared by methods known in the art, the liquid components being mixed first, followed by addition of the propellant. The amounts indicated are parts by weight.

The "lipid mixture" used in these compositions was a 60:40 blend of glycerol monolaurate and isostearyl alcohol.

The following raw materials were used in the preparation of the Examples:
- Glycerol monolaurate (GML); Monomuls 90-L12, ex BASF.
- Isostearyl alcohol (ISA); Prisorine 3515, ex Croda.
- Denatured alcohol (EtOH), ex VWR.
- Cetiol Ultimate (C11+C13), ex BASF - undecane and tridecane. A mix of C11 and C13 linear(?) alkanes.
- SiClone SR-5 (C12-16), ex Presperse - isohexadecane, isododecane, and C13-15 alkane. A mix of C12 to C16 alkanes.
- Jeechem NDA-LC (C9-13), ex Jeen - C9-13 alkane. A mix of C9 to C13 alkanes.

The compositions indicated in Tables 1, 1A, 2, 2A, and 3 and 3A were prepared by methods known the art.

The stability of these compositions was assessed following storing at 0°C for 4 weeks. Instability was manifested by crystallisation of the lipids from the composition, visually assessed. Compositions having "good" stability showed no sign of lipid crystallisation; compositions having "poor" stability did show lipid crystallisation.

The efficacy of one the compositions (N5 from Table 1) as a sweat management composition was also assessed vs. a deodorant body spray as a control. Topical application of Example N5 was found to lead to 16% reduction in sweat weight recovered compared with the deodorant control product. This result was significant at the 99% level. The test protocol used was one previously disclosed in prior publication EP 2,999,452 B1.

**Table 1: Cetiol Ultimate Pump Spray Examples**

| Compositions N1, N7, N11 and N14 of tables 1 and 1A are comparative compositions. | | | | | | |
|---|---|---|---|---|---|---|
| Components: | GML | ISA | C11+C13 alkane | Fragrance | EtOH | Stability |
| | wt. % | | | | | |
| **Example:** | | | | | | |
| **N1** | 11.06 | 7.38 | 23.56 | 1.00 | To 100 | Poor |
| **N3** | 3.65 | 2.44 | 23.56 | 1.00 | To 100 | **Good** |
| **N5** | 7.30 | 4.87 | 15.55 | 1.00 | To 100 | **Good** |
| **N7** | 11.06 | 7.38 | 7.77 | 1.00 | To 100 | Poor |
| **N9** | 3.65 | 2.44 | 7.77 | 1.00 | To 100 | **Good** |
| **N11** | 11.06 | 7.38 | 53.56 | 1.00 | To 100 | Poor |
| **N14** | 7.62 | 5.08 | 59.30 | 1.00 | To 100 | Poor |
| **N16** | 3.65 | 2.44 | 65.91 | 1.00 | To 100 | **Good** |

**Table 1A: Cetiol Ultimate Pump Spray Examples**

| Components: | GML + ISA | ISA: GML | C11+C13 alkane | EtOH + Fragrance | 0.22 x (EtOH + F) | Stability |
|---|---|---|---|---|---|---|
| **Example:** | % w/w | | % w/w | % w/w | % w/w | |
| **N1** | 18.44 | 40: 60 | 23.56 | 58.00 | 12.88 | Poor |
| **N3** | 6.09 | 40: 60 | 23.56 | 70.35 | 15.62 | **Good** |
| **N5** | 12.17 | 40: 60 | 15.55 | 72.28 | 16.05 | **Good** |
| **N7** | 18.44 | 40: 60 | 7.77 | 73.79 | 16.38 | Poor |
| **N9** | 6.09 | 40: 60 | 7.77 | 86.14 | 19.12 | **Good** |
| **N11** | 18.44 | 40: 60 | 53.56 | 28.00 | 6.22 | Poor |
| **N14** | 12.70 | 40: 60 | 59.30 | 28.00 | 6.22 | Poor |
| **N16** | 6.09 | 40: 60 | 65.91 | 28.00 | 6.22 | **Good** |

**Table 2: SiClone SR-5 Pump Spray Examples**

| Compositions N10 and N16 of tables 2 and 2A are comparative compositions. | | | | | | |
|---|---|---|---|---|---|---|
| Components: | GML | ISA | C12-C16 alkane | Fragrance | EtOH | Stability |
| | wt. % | | | | | |
| **Example:** | | | | | | |
| **N10** | 11.06 | 7.38 | 23.56 | 1.00 | To 100 | Poor |
| **N12** | 3.65 | 2.44 | 23.56 | 1.00 | To 100 | **Good** |
| **N14** | 7.30 | 4.87 | 15.55 | 1.00 | To 100 | **Good** |
| **N16** | 11.06 | 7.38 | 7.77 | 1.00 | To 100 | Poor |
| **N18** | 3.65 | 2.44 | 7.77 | 1.00 | To 100 | **Good** |

**Table 2A: SiClone SR-5 Pump Spray Examples**

| Components: | GML + ISA | ISA: GML | C12-C16 alkane | EtOH + Fragrance | 0.22 x (EtOH + F) | Stability |
|---|---|---|---|---|---|---|
| **Example:** | % w/w | | % w/w | % w/w | % w/w | |
| **N10** | 18.44 | 40: 60 | 23.56 | 58.00 | 12.88 | Poor |
| **N12** | 6.09 | 40: 60 | 23.56 | 70.35 | 15.62 | **Good** |
| **N14** | 12.17 | 40: 60 | 15.55 | 72.28 | 16.05 | **Good** |
| **N16** | 18.44 | 40: 60 | 7.77 | 73.79 | 16.38 | Poor |
| **N18** | 6.09 | 40: 60 | 7.77 | 86.14 | 19.12 | **Good** |

**Table 3: Jeechem NDA-LC Pump Spray Examples**

| Compositions N19 and N25 of tables 3 and 3A are comparative compositions. | | | | | | |
|---|---|---|---|---|---|---|
| Components: | GML | ISA | C9-C13 alkane | Fragrance | EtOH | Stability |
| | wt. % | | | | | |
| **Example:** | | | | | | |
| **N19** | 11.06 | 7.38 | 23.56 | 1.00 | To 100 | Poor |
| **N21** | 3.65 | 2.44 | 23.56 | 1.00 | To 100 | **Good** |
| **N23** | 7.30 | 7.38 | 15.55 | 1.00 | To 100 | **Good** |
| **N25** | 11.06 | 7.38 | 7.77 | 1.00 | To 100 | Poor |
| **N27** | 3.65 | 2.44 | 7.77 | 1.00 | To 100 | **Good** |

**Table 3A: Jeechem NDA-LC Pump Spray Examples**

| Components: | GML + ISA | ISA: GML | C9-C13 alkane | EtOH + Fragrance | 0.22 x (EtOH + F) | Stability |
|---|---|---|---|---|---|---|
| **Example:** | % w/w | | % w/w | % w/w | % w/w | |
| **N19** | 18.44 | 40: 60 | 23.56 | 58.00 | 12.88 | Poor |
| **N21** | 6.09 | 40: 60 | 23.56 | 70.35 | 15.62 | **Good** |
| **N23** | 12.17 | 40: 60 | 15.55 | 72.28 | 16.05 | **Good** |
| **N25** | 18.44 | 40: 60 | 7.77 | 73.79 | 16.38 | Poor |
| **N27** | 6.09 | 40: 60 | 7.77 | 86.14 | 19.12 | **Good** |

## Claims

1. A cosmetic composition comprising:
(i) 24.6 to 95% by weight ethanol;
(ii) 2.0 to 18.0% by weight of a lipid mixture consisting of isostearyl alcohol and glycerol monolaurate at a weight ratio of from 25: 75 to 40: 60;
(iii) 3 to 70% by weight of an oil selected from C8 to C16 alkanes,
wherein:
(a) the amount indicated for component (i) includes any fragrance present in the composition;
(b) the amounts indicated for components (i) to (iii) exclude any volatile propellant present in the composition and
(c) the content of component (ii) by weight is less than or equal to the content of component (i) by weight multiplied by 0.22.

2. A composition according to claim 1, wherein the lipid content of the three-component mixture is from 5% by weight.

3. A composition according to claim 1 or claim 2, wherein the ethanol content of the three-component mixture is from 10 to 45% by weight.

4. A composition according to any one of the preceding claims, wherein the propellant content of the three-component mixture is from 20 to 87.5% by weight.

5. A composition according to any one of the preceding claims, wherein the propellant comprises a blend of C3 to C4 hydrocarbons.

6. A composition according to any one of the preceding claims, wherein the content of the lipid mixture in the total composition, ignoring the propellant therein, is at least 10% by weight.

7. A composition according to claim 6, wherein the content of the lipid mixture in the total composition, ignoring the propellant therein, is at least 15% by weight.

8. A composition according to any one of the preceding claims, wherein the content of volatile silicone in the total composition, ignoring the propellant therein, is less than 5% by weight.

9. A composition according to claim 8, wherein volatile silicone is absent.

10. A composition according to any one of the preceding claims comprising a fragrance, the fragrance being considered part of the ethanol in calculating the ethanol content of the composition.

11. A composition according to any of one the preceding claims, comprising a fragrance.

12. A composition according to claim 11, wherein the fragrance is present at a level of from 0.5 to 3% by weight.

13. A composition according to any one of preceding claims, which is free from aluminium or zirconium salts.

14. A composition according to any one of preceding claims, comprising an antimicrobial agent.

15. A non-therapeutic cosmetic method of attaining a sweat management benefit comprising the topical application of a composition according to any of claims 1 to 14.

## Patentansprüche

1. Kosmetische Zusammensetzung, umfassend:
(i) 24,6 bis 95 Gewichts-% Ethanol;
(ii) 2,0 bis 18,0 Gewichts-% einer Lipidmischung, bestehend aus Isostearylalkohol und Glycerinmonolaurat in einem Gewichtsverhältnis von 25:75 bis 40:60;
(iii) 3 bis 70 Gewichts-% eines Öls, ausgewählt unter C8- bis C16-Alkanen,
wobei
(a) die für die Komponente (i) angegebene Menge alle in der Zusammensetzung vorhandenen Duftstoffe einschließt;
(b) die für die Komponenten (i) bis (iii) angegebenen Mengen alle in der Zusammensetzung vorhandenen flüchtigen Treibmittel ausschließen und
(c) der Gewichtsgehalt der Komponente (ii) kleiner als oder gleich dem Gewichtsgehalt der Komponente (i) multipliziert mit 0,22 ist.

2. Zusammensetzung nach Anspruch 1, wobei der Lipidgehalt der Dreikomponentenmischung ab 5 Gewichts-% beträgt.

3. Zusammensetzung nach Anspruch 1 oder Anspruch 2, wobei der Ethanolgehalt der Dreikomponentenmischung 10 bis 45 Gewichts-% beträgt.

4. Zusammensetzung nach irgendeinem der vorhergehenden Ansprüche, wobei der Treibmittelgehalt der Dreikomponentenmischung 20 bis 87,5 Gewichts-% beträgt.

5. Zusammensetzung nach irgendeinem der vorhergehenden Ansprüche, wobei das Treibmittel eine Mischung aus C3- bis C4-Kohlenwasserstoffen umfasst.

6. Zusammensetzung nach irgendeinem der vorhergehenden Ansprüche, wobei der Gehalt der Lipidmischung in der Gesamtzusammensetzung, das darin enthaltene Treibmittel außer Betracht lassend, mindestens 10 Gewichts-% beträgt.

7. Zusammensetzung nach Anspruch 6, wobei der Gehalt der Lipidmischung in der Gesamtzusammensetzung, das darin enthaltene Treibmittel außer Betracht lassend, mindestens 15 Gewichts-% beträgt.

8. Zusammensetzung nach irgendeinem der vorhergehenden Ansprüche, wobei der Gehalt des flüchtigen Silikons in der Gesamtzusammensetzung, das darin enthaltene Treibmittel außer Betracht lassend, weniger als 5 Gewichts-% beträgt.

9. Zusammensetzung nach Anspruch 8, in der kein flüchtiges Silikon vorhanden ist.

10. Zusammensetzung nach irgendeinem der vorhergehenden Ansprüche, die einen Duftstoff umfasst, wobei der Duftstoff bei der Berechnung des Ethanolgehalts der Zusammensetzung als Teil des Ethanols betrachtet wird.

11. Zusammensetzung nach irgendeinem der vorhergehenden Ansprüche, umfassend einen Duftstoff.

12. Zusammensetzung nach Anspruch 11, wobei der Duftstoff in einer Konzentration von 0,5 bis 3 Gewichts-% vorliegt.

13. Zusammensetzung nach irgendeinem der vorhergehenden Ansprüche, die frei von Aluminium- oder Zirkonium-Salzen ist.

14. Zusammensetzung nach irgendeinem der vorhergehenden Ansprüche, umfassend ein antimikrobielles Mittel.

15. Nicht-therapeutisches kosmetisches Verfahren zur Erzielung des Vorteils einer Schweißregulierung, die die topische Anwendung einer Zusammensetzung nach einem der Ansprüche 1 bis 14 umfasst.

## Revendications

1. Composition cosmétique comprenant :
(i) 24,6 à 95 % en poids d'éthanol ;
(ii) 2,0 à 18,0 % en poids d'un mélange lipidique se composant d'alcool isostéarylique et de monolaurate de glycérol à un rapport en poids de 25:75 à 40:60 ;
(iii) 3 à 70 % en poids d'une huile choisie parmi les alcanes en C8 à C16, dans laquelle :
(a) la quantité indiquée pour le composant (i) comprend tout parfum présent dans la composition ;
(b) les quantités indiquées pour les composants (i) à (iii) excluent tout propulseur volatil présent dans la composition et
(c) la teneur du composant (ii) en poids est inférieure ou égale à la teneur du composant (i) en poids multipliée par 0,22.

2. Composition selon la revendication 1, dans laquelle la teneur en lipide du mélange à trois composants est à partir de 5 % en poids.

3. Composition selon la revendication 1 ou la revendication 2, dans laquelle la teneur en éthanol du mélange à trois composants va de 10 à 45 % en poids.

4. Composition selon l'une quelconque des revendications précédentes, dans laquelle la teneur en propulseur du mélange à trois composants va de 20 à 87,5 % en poids.

5. Composition selon l'une quelconque des revendications précédentes, dans laquelle le propulseur comprend un mélange d'hydrocarbures en C3 à C4.

6. Composition selon l'une quelconque des revendications précédentes, dans laquelle la teneur du mélange lipidique dans la composition totale, à l'exclusion du propulseur dans celle-ci, est au moins de 10 % en poids.

7. Composition selon la revendication 6, dans laquelle la teneur du mélange lipidique dans la composition totale, à l'exclusion du propulseur dans celle-ci, est au moins de 15 % en poids.

8. Composition selon l'une quelconque des revendications précédentes, dans laquelle la teneur en silicone volatile dans la composition totale, à l'exclusion du propulseur dans celle-ci, est inférieure à 5 % en poids.

9. Composition selon la revendication 8, dans laquelle la silicone volatile est absente.

10. Composition selon l'une quelconque des revendications précédentes comprenant un parfum, le parfum étant une partie considérée de l'éthanol dans le calcul de la teneur en éthanol de la composition.

11. Composition selon l'une quelconque des revendications précédentes, comprenant un parfum.

12. Composition selon la revendication 11, dans laquelle le parfum est présent à un taux allant de 0,5 à 3 % en poids.

13. Composition selon l'une quelconque des revendications précédentes, qui est dépourvue de sels d'aluminium ou de zirconium.

14. Composition selon l'une quelconque des revendications précédentes, comprenant un agent antimicrobien.

15. Procédé cosmétique non thérapeutique permettant d'obtenir un bénéfice de gestion de la transpiration comprenant l'application topique d'une composition selon l'une quelconque des revendications 1 à 14.
